# FASCICULE DE BREVET EUROPEEN

(11) **EP 0 499 550 B1**
(45) Date de publication et mention de la délivrance du brevet: **07.05.1997**
(21) Numéro de dépôt: 92420032.2
(22) Date de dépôt: 27.01.1992
(51) Int. Cl.: G01N 33/12, A22B 5/00

(54) **Procédé pour le classement de carcasses de gros bovins, veaux, porcs ou ovins et dispositif pour sa mise en oeuvre**
Verfahren und Vorrichtung zur Klassifizierung von Grossvieh, Kälbern, Schweinen oder Schafen
Method and means for classifying carcasses of cattle, calves, swine or sheep

(30) Priorité: 14.02.1991 FR 9102297
(43) Date de publication de la demande: 19.08.1992
(73) Titulaire: NORMACLASS, F-75116 Paris (FR)
(72) Inventeur: Chevalier, Patrick, F-28120 Olle (FR); Villemin, Michèle, F-94490 Ormesson sur Marne (FR); Plusa, Janusz, F-94370 Sucy en Brie (FR); Leclere, Jean, F-94000 Creteil (FR)
(74) Mandataire: Maureau, Philippe

(56) Documents cités:
- EP-A- 0 046 704
- EP-A- 0 321 981
- EP-A- 0 361 625
- DE-A- 3 049 589
- DK-A- 6 764
- FR-A- 2 462 205
- FR-A- 2 488 530
- FR-A- 2 545 010
- FR-A- 2 608 899
- GB-A- 2 000 280

## Description

La présente invention a pour objet un procédé pour le classement de carcasses de gros bovins, veaux, porcs ou ovins, et un dispositif pour sa mise en oeuvre.

Il existe des différences importantes au niveau de la morphologie et de l'état d'engraissement des animaux d'une même espèce. Il convient donc, après abattage des animaux, de réaliser le classement des carcasses afin de déterminer, en fonction de la qualité de chacune d'elles, d'une part, le prix qui en sera payé à l'éleveur en amont, d'autre part, l'utilisation qui en sera faite en aval.

Ce classement intervient à la fin des chaînes d'abattage, c'est-à-dire après éviscération et préparation des animaux, sur des carcasses entières (veaux, ovins) où sur des demi-carcasses (gros bovins, porcs) suspendues à une chaîne d'abattage avançant de façon continue ou par pas.

Dans le cas des gros bovins, deux critères indépendants ont été retenus pour réaliser le classement des carcasses : la conformation et l'état d'engraissement. Une grille descriptive communautaire, basée sur l'appréciation visuelle de ces deux critères, a été officialisée par les règlements CEE du 28.04.81, du 12. 12.10.81.

La conformation tient compte de la quantité de viande présente sur la carcasse, en fonction des dimensions de celle-ci. La conformation se détermine par l'appréciation des profils et des épaisseurs musculaires. Cinq classes ont été définies, chacune étant caractérisée par l'une des lettres du mot EUROP. La qualité de la conformation décroît de la classe E, qui correspond à une carcasse de conformation excellente avec des profils convexes et un excellent développement musculaire, jusqu'à la classe P qui correspond à une conformation médiocre, avec des profils concaves et un développement musculaire très réduit.

L'état d'engraissement s'apprécie en examinant la graisse de couverture et la graisse à l'intérieur de la cage thoracique. Cinq classes ont été définies, chacune étant caractérisée par un chiffre de 1 pour un engraissement "très faible", à 5 pour un engraissement "très fort".

Chaque classe de conformation et d'état d'engraissement peut être subdivisée en 3 sous-classes (+, =, -) pour améliorer la précision du classement.

Actuellement, le classement des carcasses est réalisé par des opérateurs qui visualisent chaque carcasse et lui attribuent une classe ou sous-classe de conformation ainsi qu'une classe ou sous-classe d'état d'engraissement. Il faut toutefois considérer que ce mode de classement, bien que se référant à une grille officielle, présente un caractère subjectif puisqu'il dépend uniquement de l'appréciation visuelle faite par un opérateur soumis à des conditions variables de travail. Ceci entraîne des variations dans le classement d'une même carcasse par des opérateurs différents et dans des abattoirs différents.

Pour remédier à ces inconvénients, il a été imaginé d'utiliser des appareils susceptibles de fournir un classement, toujours selon la grille communautaire EUROP, qui soit moins subjectif et surtout uniforme dans le temps et sur les différents sites d'abattage.

A cet effet, il a été imaginé de réaliser des mesures de façon mécanique. La solution décrite dans les brevets français AUGE (79 19310, 80 17947), qui est limitée à l'appréciation du classement en conformation, ne donne pas satisfaction du fait qu'il faut immobiliser chaque carcasse à classer, amener à son contact un certain nombre d'éléments de mesure tels que des palpeurs, lire les mesures et enfin la libérer des organes de mesure, ce qui peut être gênant pour suivre des cadences élevées.

Il existe également des dispositifs permettant de mesurer l'état d'engraissement, mettant en oeuvre une sonde qui doit être enfoncée dans les tissus en des sites déterminés de la carcasse et qui donne une mesure des épaisseurs de gras et de muscles traversées par la sonde (FAT-O-METER de SKF, HENNESSY). La mise en oeuvre manuelle est coûteuse et est incompatible avec des cadences élevées. La mise en oeuvre automatique est difficile à réaliser sachant que toutes les carcasses ne possèdent pas la même taille, et qu'elles avancent le long de la chaîne d'abattage.

Il existe des dispositifs de classement utilisant l'analyse d'images vidéo (PFISTER-SKG ; PETERSEN demande de brevet danois 6764/87) qui permettent de donner un classement en conformation par analyse du contour de certaines parties de la carcasse et un classement en engraissement par analyse du contraste observé entre gras et muscle. Ce dispositif étudié chez le porc pour donner une estimation de rendement en viande ne donne pas de résultats satisfaisants par rapport à la mesure réelle de rendement. Chez le gros bovin dont la carcasse est plus complexe que celle du porc, le dispositif utilisé sous forme d'une cabine contenant une seule caméra ne permet pas de donner avec assez de précision le classement selon la grille communautaire EUROP et oblige à utiliser une sonde manuelle pour compléter les mesures vidéo.

Le document DE-A-3 049 589 concerne un procédé pour le classement de carcasses de porcs consistant à réaliser la mise en appui d'une demi-carcasse, par sa face longitudinale, sur un support, à réaliser un positionnement angulaire de celle-ci pour procéder à des prises de vue optiques sous différents angles, à stocker celles-ci dans la mémoire d'un calculateur, et à traiter les informations saisies pour réaliser le classement de la carcasse.

Les caractéristiques techniques de ce procédé n'autorisent pas un positionnement suffisamment régulier et référencé, ni une extraction de contours correcte, elles ne permettent donc pas de déterminer avec suffisamment de précision le pourcentage de chair de l'ensemble de la carcasse, ni de classer suivant les grilles communautaires les carcasses des différentes espèces.

Le but de l'invention est de fournir un procédé et un dispositif permettant de réaliser de façon automatique et par mise en oeuvre de moyens qui ne viennent pas en contact physique avec les carcasses, le classement de carcasses d'animaux, qui puissent être installés sur la chaîne d'abattage après pesée, qui fournissent les informations à partir de l'analyse d'une demi-carcasse ou d'une carcasse entière quels que soient la taille, le poids, la forme et la race de la carcasse considérée, et qui puissent fournir le classement d'environ cent carcasses à l'heure en conformation et en état d'engraissement.

A cet effet, ce procédé, du type précité, consiste :
- à amener la demi-carcasse en appui, d'une part, par sa face longitudinale contre une surface-support située dans un plan sensiblement vertical correspondant sensiblement au plan vertical contenant les crochets de suspension de la chaîne d'abattage, cette surface support étant constituée par un tapis sans fin entraîné à même vitesse que la chaîne et, d'autre part, par sa face dorsale, à proximité de son extrémité inférieure, contre une seconde surface-support, disposée à l'extrémité aval de celle-ci, dans le sens d'avancement de la chaîne d'abattage, constituée par un volet pivotant perpendiculaire au tapis,
- à positionner en hauteur avec précision l'ensemble tapis-volet grâce à une caméra (L),
- à arrêter la chaîne après que la demi-carcasse soit venue prendre appui sur le volet,
- à réaliser un positionnement angulaire de la carcasse pour procéder à des prises de vue optiques sous différents angles au niveau de l'arrière-train, de la cuisse, de la partie haute de l'échine et de la partie avant de la demi-carcasse correspondant à la zone basse de celle-ci,
- à stocker les prises de vue dans la mémoire d'un calculateur, qui contient déjà les mesures de poids et de longueur de cette demi-carcasse,
- à traiter les informations saisies en tenant compte d'informations théoriques pour réaliser le classement des carcasses,
- et à faire basculer le volet vers sa position escamotée pour libérer la carcasse et permettre son évacuation.

Les informations théoriques résultent de la synthèse d'informations recueillies préalablement en grand nombre et traitées statistiquement par rapport au classement de référence en conformation EUROP, ce qui permet de déterminer de façon parfaitement fidèle le classement de chaque carcasse par rapport à la grille EUROP.

Selon une autre caractéristique de ce procédé, celui-ci consiste à réaliser au moins une prise de vue sur la face interne de la carcasse, à envoyer cette information au calculateur et à traiter cette information en tenant compte d'informations recueillies préalablement en grand nombre et traitées statistiquement par rapport au classement en état d'engraissement selon la grille communautaire. Ce traitement permet de déterminer de façon parfaitement fidèle le classement en état d'engraissement selon la grille communautaire.

Avantageusement, ce procédé consiste à effectuer tout d'abord la pesée de chaque demi-carcasse dont le classement est à réaliser, le poids mesuré donnant une information sur la longueur de la carcasse, qui est utilisée pour effectuer un premier réglage de position en hauteur du support d'appui de la demi-carcasse, puis à effectuer une mesure optique de la longueur de celle-ci, qui fournit une information utilisée pour effectuer un réglage précis de la position en hauteur du support d'appui de la demi-carcasse.

Selon un premier mode de mise en oeuvre de ce procédé, celui-ci consiste à réaliser un éclairage direct de la carcasse, du côté de la prise de vue avec réalisation d'un contraste entre la carcasse et le fond sur lequel se détache celle-ci.

Selon un autre mode de mise en oeuvre de ce procédé, celui-ci consiste à réaliser un éclairage indirect de la carcasse, grâce à un fond lumineux assurant un éclairage diffus de toute la carcasse.

Le but recherché dans l'un et l'autre cas est d'obtenir une parfaite définition des contours de la carcasse vis-à-vis du fond sur lequel celle-ci se détache.

Un dispositif pour la mise en oeuvre de ce procédé comprend :
- un système d'appui pour la demi-carcasse se présentant sous la forme d'un dièdre délimité par deux surfaces séparées l'une de l'autre par une arête sensiblement verticale, l'une des faces du dièdre destinées à prendre appui contre la face coupée de la demi-carcasse étant constituée par un tapis sans fin, monté sur des axes verticaux, avec l'une des faces du tapis disposée sensiblement dans le plan des crochets de suspension de la chaîne d'abattage et entraîné en synchronisme avec cette dernière, et l'autre face du dièdre destinée à prendre appui contre la face dorsale de la demi-carcasse, à proximité de la partie inférieure de cette dernière, étant située à proximité de l'extrémité aval du tapis, dans le sens d'avancement de la chaîne d'abattage, étant constitué par un volet disposé dans un plan vertical, monté pivotant entre une position dans laquelle il est perpendiculaire au brin de la face active du tapis, et une position dans laquelle il est sensiblement dans le prolongement de celui-ci, le dièdre étant monté pivotant autour d'un axe vertical correspondant à l'axe du crochet et associé à des moyens d'entraînement en rotation assurant successivement son positionnement dans plusieurs positions angulaires prédéterminées correspondant aux différents angles de prise de vue,
- des caméras montées sur un support fixe et destinées à prendre des vues de la demi-carcasse au niveau de l'arrière-train, de la cuisse, de la partie haute de l'échine et de sa partie avant,
- un calculateur destiné à stocker les prises de vue, ainsi que les mesures de longueur et de poids de la demi-carcasse, et à traiter les informations saisies en tenant compte d'informations théoriques pour réaliser le classement des carcasses,
- des moyens d'escamotage de la seconde face du dièdre, pour libérer la carcasse et permettre son évacuation.

En outre, le tapis est équipé d'un système de lavage, et le support du tapis est monté pivotant autour d'un axe vertical.

En pratique, le dièdre est introduit dans le plan de déplacement des demi-carcasses sur la chaîne, sert au positionnement d'une demi-carcasse, assure la mise en pivotement de cette demi-carcasse pour lui faire occuper successivement plusieurs positions angulaires correspondant aux différents angles de prise de vue, ramène la demi-carcasse dans sa position d'origine, puis est escamoté afin de libérer la demi-carcasse et de permettre la poursuite de son cheminement sur la chaîne d'abattage.

Selon une autre forme d'exécution de ce dispositif, le dièdre est de hauteur correspondant sensiblement à la distance entre le sol et la chaîne transportant les carcasses. Ce fond lumineux assure un éclairage indirect de la demi-carcasse, pour les appareils de prise de vue disposés de l'autre côté de la chaîne d'abattage.

Selon une autre caractéristique de l'invention, le mouvement de déplacement vertical du dièdre destiné à réaliser l'appui d'une demi-carcasse, est asservi d'une part, à un dispositif de pesage en vue d'un réglage grossier et, d'autre part, à un dispositif optique de la mesure de la longueur de la carcasse, situé en amont du dispositif en vue d'un réglage fin, de telle sorte que le dièdre prenne appui sur la partie du dos de l'animal située au-dessus du collier de celui-ci. Il convient en effet de réaliser un appui sur une partie rigide et sur une surface plane. Il convient donc de réaliser cet appui au-dessus du collier.

Conformément à une autre caractéristique de l'invention, l'angle de rotation du dièdre est de l'ordre de 70 degrés par rapport à la position d'origine, et les prises de vue sont réalisées par au moins trois caméras, dont l'axe optique de la première est perpendiculaire à la direction de la chaîne d'abattage et dont les deux autres caméras, dont les axes optiques sont contenus dans un plan horizontal, forment un angle de 45 degrés, et sont disposées symétriquement par rapport à la première caméra.

Avantageusement, le support du dièdre est équipé d'une caméra qui est destinée à prendre des vues de la partie interne de la demi-carcasse, cette caméra étant associée à un dispositif d'éclairage additionnel et asservi au dispositif de mesure de la longueur de la carcasse.

De toute façon, l'invention sera bien comprise à l'aide de la description qui suit, en référence au dessin schématique annexé représentant, à titre d'exemple non limitatif, une forme d'exécution de ce dispositif ;
Figure 1 est une vue très schématique de l'implantation des différents éléments constitutifs de ce dispositif ;
Figure 2 en est une vue de dessus ;
Figures 3 et 4 sont deux vues respectivement de dessus, partiellement en coupe horizontale et de côté du dispositif assurant la retenue et le positionnement de chaque demi-carcasse au cours de la saisie des formes.

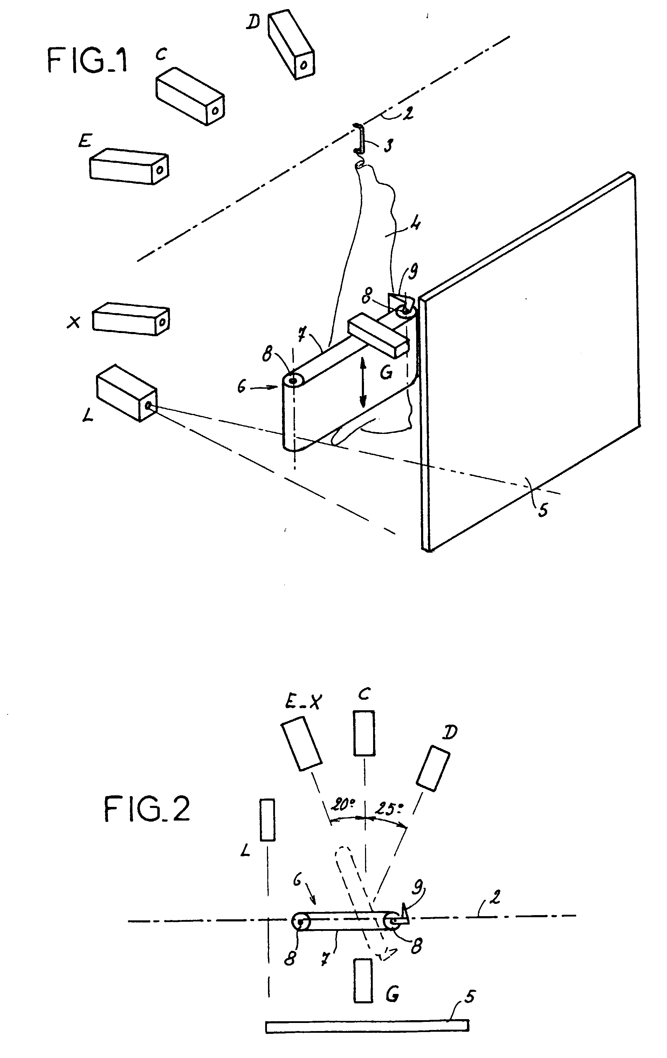

Le dispositif représenté à la figure 1 montre une chaîne désignée par la référence générale 2 à laquelle sont fixés des crochets 3, dont l'un est représenté au dessin, servant chacun à la suspension d'une demi-carcasse 4.

Cette chaîne n'est pas décrite en détail, étant de type connu, et par exemple animée d'un mouvement d'avance par pas.

Du côté de la demi-carcasse correspondant à la face coupée de celle-ci, est disposé un élément 5 diffuseur de lumière, tel qu'un caisson contenant des sources lumineuses, dont la face, située du côté de la chaîne, est délimitée par un verre dépoli. La hauteur de cet écran est importante, et correspond sensiblement à la hauteur des demi-carcasses dont le classement est à réaliser.

Du côté opposé de la chaîne 2, sont montées sur des supports fixes un certain nombre de caméras. Une première caméra L située en amont de l'écran 5 est destinée à assurer la mesure de la longueur de la carcasse. Une caméra C est disposée en regard de l'écran 5, dont l'axe optique est perpendiculaire à la direction de la chaîne. Deux autres caméras D et E sont disposées de telle sorte que les axes optiques contenus dans un plan horizontal forment un angle de 45 degrés, et forment des angles respectifs et approximativement symétriques de 25 degrés et 20 degrés par rapport à l'axe de la caméra C. Les caméras C, D et E sont destinées à prendre des vues de la demi-carcasse au niveau de l'arrière-train, de la cuisse et du haut de l'échine. Une caméra X située dans le même plan vertical que la caméra E est destinée à réaliser une prise de vue de la partie basse de la demi-carcasse correspondant à l'avant de cette dernière.

Du côté de l'écran 5, et entre celui-ci et la chaîne 2, est disposé un système de positionnement de chaque demi-carcasse.

Ce système désigné par la référence générale 6, comprend un tapis sans fin 7 monté sur deux rouleaux 8 d'axes verticaux, ainsi qu'un volet 9 pivotant vis-à-vis du tapis 7 entre une position dans laquelle il est perpendiculaire au tapis et une position dans laquelle il est sensiblement dans l'alignement de celui-ci. Le système comprend également une caméra G destinée à réaliser des prises de vue des côtes de la demi-carcasse près de la hampe, cette caméra G étant équipée de projecteurs d'éclairage additionnel et d'un système de lavage.

Il est également possible de prévoir des caméras non représentées au dessin, d'axes optiques parallèles à ceux des caméras C, D ou E fonctionnant avec un éclairage additionnel destiné à réaliser l'analyse de la couverture en gras de la face extérieure de la demi-carcasse considérée.

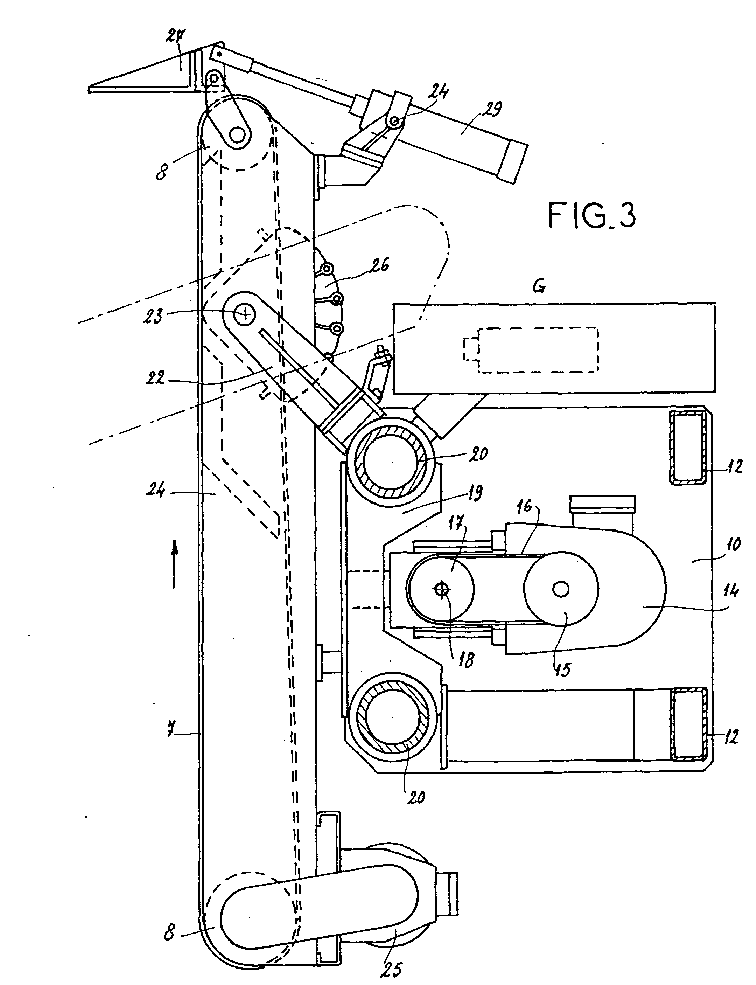

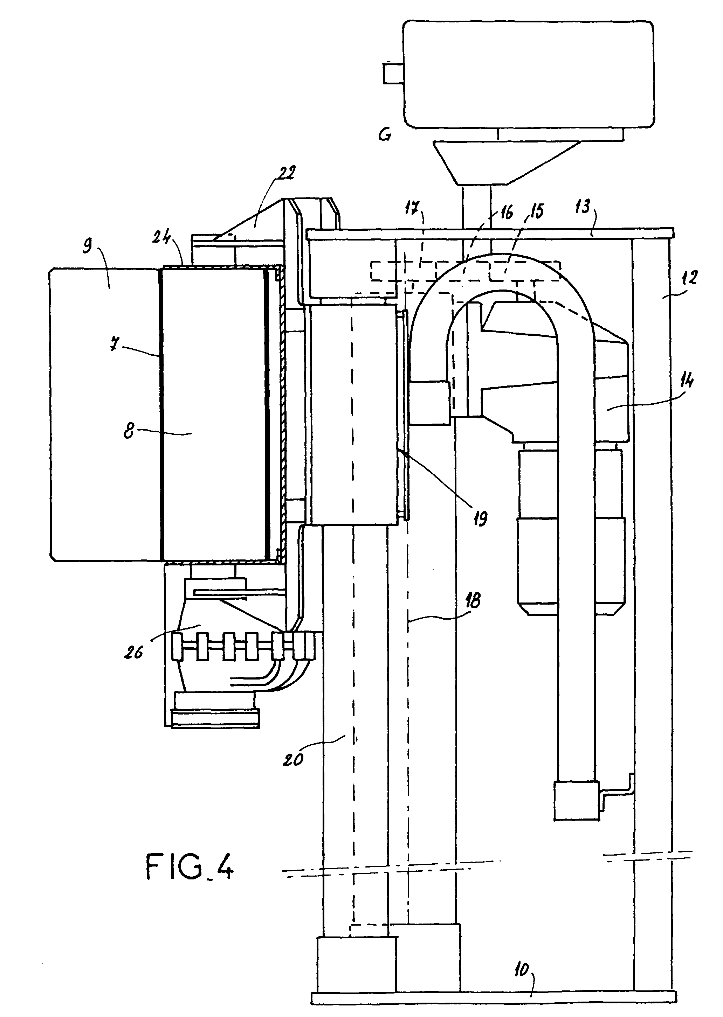

Les figures 3 et 4 montrent plus en détail le dispositif de positionnement de chaque demi-carcasse à analyser.

Ce dispositif comprend une platine 10 portant un bâti constitué, notamment, par des profilés verticaux 12, et une platine supérieure 13. Sur le bâti est fixé un moteur 14 dont l'arbre de sortie'est équipé d'une poulie 15 entraînant par une courroie 16 une poulie émettrice 17, agissant sur une vis à billes dont l'axe 18 est représenté au dessin. Cette vis à billes entraîne un coulisseau 19 monté déplaçable verticalement sur deux colonnes 20.

Sur le coulisseau 19 est fixé un bras 22, à l'extrémité libre duquel est monté pivotant autour d'un axe horizontal 23, un carter 24. Ce carter 24 porte des paliers pour les deux rouleaux 8 servant au guidage du tapis 7. L'entraînement d'un rouleau 8 est réalisé par un moteur 25 en synchronisme avec le mouvement de la chaîne 2.

Le carter est animé d'un mouvement de rotation autour de son axe 23, par l'intermédiaire d'un vérin rotatif 26. Le carter 24 porte également un volet 27 vertical, monté pivotant autour de l'axe d'un rouleau 8 entre une position, telle que représentée à la figure 3 dans laquelle il est perpendiculaire au tapis, et une position dans laquelle il est situé sensiblement dans le prolongement de celui-ci. Ce mouvement de pivotement du volet est réalisé à l'aide d'un vérin 29, dont le corps est monté articulé autour d'un axe vertical sur le carter 24, et dont l'extrémité libre de la tige est articulée également autour d'un axe vertical sur le volet 9.

Le fonctionnement de ce dispositif est le suivant :

Un dispositif de pesage, de type connu, associé à la chaîne, fournit au calculateur l'information du poids de chaque demi-carcasse à classer. Compte tenu de la relation entre le poids et la longueur de la demi-carcasse, le calculateur commande un positionnement approximatif de l'ensemble constitué par le tapis 7, le volet 27 et la caméra G.

La caméra L permet la détermination de la longueur exacte de la demi-carcasse à analyser. En fonction de cette longueur, il est procédé au positionnement précis en hauteur de l'ensemble mobile constitué par le tapis 7, le volet 27 et la caméra G. Le tapis 7 étant entraîné à la même vitesse que la chaîne 2, la demi-carcasse vient en contact avec le tapis, jusqu'à prendre appui contre le volet 27. A ce moment, la chaîne et le tapis étant arrêtés, il est procédé à la rotation de l'ensemble pivotant, tapis 7 et volet 27, autour de l'axe 23 d'un angle de l'ordre de 70 degrés. Il est alors procédé aux prises de vue à l'aide des caméras E, D et éventuellement X, après quoi, l'ensemble pivotant revient dans sa position d'origine pour réaliser les prises de vue à l'aide des caméras C et G. Les prises de vue étant terminées, le volet 27 est basculé vers sa position escamotée, assurant la libération de la demi-carcasse qui est évacuée par la chaîne 2. Les informations résultant des saisies optiques, auxquelles s'ajoutent les informations sur la longueur de la demi-carcasse et sur le poids de celle-ci sont alors traitées pour déterminer la conformation de la demi-carcasse, et son état d'engraissement.

Comme il ressort de ce qui précède, l'invention apporte une grande amélioration à la technique existante, en fournissant un procédé et un dispositif permettant le classement de carcasses d'animaux en conformation et état d'engraissement selon la grille EUROP de façon fidèle, à des cadences élevées, sur la chaîne même d'abattage.

Comme il va de soi, l'invention ne se limite pas à la seule forme d'exécution de ce dispositif, décrite ci-dessus à titre d'exemple, elle en embrasse au contraire toutes les variantes de réalisation. C'est ainsi, notamment, que le système de positionnement de la carcasse pourrait être différent et constitué, par exemple, par un dièdre délimité par deux surfaces fixes l'une relativement à l'autre, ou encore que la mise en contraste de la carcasse vis-à-vis de son fond pourrait être obtenue non pas par un éclairage indirect, à l'aide d'un verre dépoli, mais par un éclairage direct, par des projecteurs situés du côté des caméras, afin que la carcasse se découpe sur un fond contrasté.

Le dispositif peut être conçu pour traiter une demi-carcasse ou les deux demi-carcasses obtenues à partir d'une même carcasse.

Pour la détermination à la fois de la conformation et de l'état d'engraissement, il est possible d'utiliser soit des caméras distinctes, soit des caméras associées à deux modes d'éclairage différents. Il est également possible d'agencer le dièdre de telle sorte que la surface de celui-ci parallèle à la direction de la chaîne ne soit pas verticale, mais inclinée d'un angle compris entre 0 et 20 degrés par rapport à la verticale, par exemple 7 degrés. Cette surface est inclinée de haut en bas et de l'extérieur vers l'intérieure, de telle sorte que ses bords soient disposés de part et d'autre du plan vertical contenant la chaîne. On a ainsi la certitude que la demi-carcasse prenne parfaitement appui dans le dièdre et soit à coup sûr immobilisée. Compte tenu de cette inclinaison, il est possible de prévoir en amont du dièdre une glissière amenant progressivement la demi-carcasse dans la position d'inclinaison du dièdre, lors de l'avance de la chaîne.

## Revendications

1. Procédé pour le classement de carcasses de gros bovins, veaux, porcs ou ovins, consistant à mettre en appui une demi-carcasse, par la face longitudinale de celle-ci correspondant à l'axe de symétrie de la carcasse, contre un support, à procéder à des prises de vue optiques sous différents angles, à stocker ces prises de vue dans la mémoire d'un calculateur et à traiter les informations saisies pour réaliser le classement de la carcasse, caractérisé en ce qu'il consiste :
- à amener la demi-carcasse en appui, d'une part, par sa face longitudinale contre une surface-support située dans un plan sensiblement vertical correspondant sensiblement au plan vertical contenant les crochets de suspension de la chaîne d'abattage, cette surface support étant constituée par un tapis sans fin entraîné à même vitesse que la chaîne et, d'autre part, par sa face dorsale, à proximité de son extrémité inférieure, contre une seconde surface-support, disposée à l'extrémité aval de celle-ci, dans le sens d'avancement de la chaîne d'abattage, constituée par un volet pivotant perpendiculaire au tapis,
- à positionner en hauteur avec précision l'ensemble tapis-volet grâce à une caméra (L),
- à arrêter la chaîne après que la demi-carcasse soit venue prendre appui sur le volet,
- à réaliser un positionnement angulaire de la carcasse pour procéder à des prises de vue optiques sous différents angles au niveau de l'arrière-train, de la cuisse, de la partie haute de l'échine et de la partie avant de la demi-carcasse correspondant à la zone basse de celle-ci,
- à stocker les prises de vue dans la mémoire d'un calculateur, qui contient déjà les mesures de poids et de longueur de cette demi-carcasse,
- à traiter les informations saisies en tenant compte d'informations théoriques pour réaliser le classement des carcasses,
- et à faire basculer le volet vers sa position escamotée pour libérer la carcasse et permettre son évacuation.

2. Procédé selon la revendication 1, caractérisé en ce qu'il consiste à réaliser au moins une prise de vue sur la face intérieure de la carcasse, à envoyer cette information au calculateur, et à traiter cette information en fonction d'éléments mémorisés en vue de déterminer l'état d'enregistrement.

3. Procédé selon l'une quelconque des revendications 1 et 2, caractérisé en ce qu'il consiste à effectuer tout d'abord la pesée de chaque demi-carcasse dont le classement est à réaliser, le poids mesuré donnant une information sur la longueur de la carcasse, qui est utilisée pour effectuer un premier réglage de la position en hauteur du support d'appui de la demi-carcasse.

4. Procédé selon la revendication 3, caractérisé en ce qu'il consiste, après pesée de chaque demi-carcasse, à effectuer une mesure optique de la longueur de celle-ci, qui fournit une information utilisée pour effectuer un réglage précis de la position en hauteur du support d'appui de la demi-carcasse.

5. Procédé selon la revendication 1, caractérisé en ce qu'il consiste à réaliser un éclairage direct de la carcasse (4) du côté de la prise de vue, ce qui permet d'obtenir un contraste entre la carcasse et le fond sur lequel se détache celle-ci.

6. Procédé selon la revendication 1, caractérisé en ce qu'il consiste à réaliser un éclairage indirect de la carcasse, grâce à un fond lumineux assurant un éclairage diffus de toute la carcasse.

7. Dispositif pour la mise en oeuvre du procédé selon la revendication 1, caractérisé en ce qu'il comprend :
- un système d'appui pour la demi-carcasse (4) se présentant sous la forme d'un dièdre délimité par deux surfaces (7, 9) séparées l'une de l'autre par une arête sensiblement verticale, l'une (7) des faces du dièdre destinée à prendre appui contre la face coupée de la demi-carcasse étant constituée par un tapis sans fin (7), monté sur des axes verticaux (8), avec l'une des faces du tapis disposée sensiblement dans le plan des crochets de suspension (3) de la chaîne d'abattage et entraîné en synchronisme avec cette dernière, et l'autre face (9) du dièdre destinée à prendre appui contre la face dorsale de la demi-carcasse, à proximité de la partie inférieure de cette dernière, étant située à proximité de l'extrémité aval du tapis (7), dans le sens d'avancement de la chaîne d'abattage, étant constitué par un volet (27) disposé dans un plan vertical, monté pivotant entre une position dans laquelle il est perpendiculaire au brin de la face active du tapis, et une position dans laquelle il est sensiblement dans le prolongement de celui-ci, le dièdre étant monté pivotant autour d'un axe vertical correspondant à l'axe du crochet et associé à des moyens d'entraînement en rotation assurant successivement son positionnement dans plusieurs positions angulaires prédéterminées correspondant aux différents angles de prise de vue,
- une caméra (L) pour positionner en hauteur avec précision l'ensemble tapis-volet
- des caméras (C, D, E, G, X) montées sur un support fixe et destinées à prendre des vues de la demi-carcasse au niveau de l'arrière-train, de la cuisse, de la partie haute de l'échine et de sa partie avant,
- un calculateur destiné à stocker les prises de vue, ainsi que les mesures de longueur et de poids de la demi-carcasse, et à traiter les informations saisies en tenant compte d'informations théoriques pour réaliser le classement des carcasses,
- des moyens d'escamotage de la seconde face (9) du dièdre, pour libérer la carcasse et permettre son évacuation.

8. Dispositif selon la revendication 7, caractérisé en ce que le tapis est équipé d'un système de lavage, et le support du tapis est monté pivotant autour d'un axe vertical (23).

9. Dispositif selon la revendication 7, caractérisé en ce que la largeur du tapis (7) c'est-à-dire la dimension de celui-ci considérée dans le sens de la hauteur, est sensiblement inférieure à la longueur de la carcasse (4), et le support (24) du tapis est monté déplaçable verticalement.

10. Dispositif selon la revendication 9, caractérisé en ce que les moyens de déplacement vertical du support (24) de tapis (7) et du volet (27) qui lui est associé sont constitués par un coulisseau (19), sur lequel est monté de façon articulée le support du tapis et du volet, ce coulisseau étant lui-même monté sur des colonnes verticales (20), avec entraînement vertical par l'intermédiaire d'une vis à billes.

11. Dispositif selon la revendication 7, caractérisé en ce que le dièdre constitué par le tapis (7) et le volet (9, 27), est disposé en avant d'un panneau lumineux (5) de hauteur correspondant sensiblement à la distance entre le sol et la chaîne transportant les carcasses.

12. Dispositif selon l'une quelconque des revendications 7 à 11, caractérisé en ce que le mouvement de déplacement vertical du dièdre destiné à réaliser l'appui d'une demi-carcasse, est asservi, d'une part, à un dispositif de pesage de la demi-carcasse disposé en amont pour un réglage grossier et, d'autre part, à un dispositif optique de la mesure de la longueur de la carcasse également disposé en amont du dièdre, pour un réglage fin, de telle sorte que le dièdre prenne appui sur la partie du dos de l'animal située au-dessus du collier de celui-ci.

13. Dispositif selon l'une quelconque des revendications 7 à 12, caractérisé en ce que l'angle de rotation du dièdre est de l'ordre de 70 degrés par rapport à la position d'origine, et les prises de vue sont réalisées par au moins trois caméras, dont l'axe optique de la première (C) est perpendiculaire à la direction de la chaîne d'abattage (2) et dont les deux autres caméras (D, E) dont les axes optiques sont contenus dans un plan horizontal, forment un angle de 45°, et sont disposés symétriquement par rapport à la première caméra (C).

14. Dispositif selon l'une quelconque des revendications 7 à 13, caractérisé en ce que le support du dièdre est équipé d'une caméra (G) qui est destinée à prendre des vues de la partie interne de la demi-carcasse, cette caméra étant associée à un dispositif d'éclairage additionnel, et asservi au dispositif de mesure de la longueur de la carcasse.

15. Dispositif selon l'une quelconque des revendications 13 à 14, caractérisé en ce qu'il comprend des caméras d'axes optiques parallèles à ceux des caméras fixes C, D ou E, fonctionnant avec éclairage additionnel, pour analyser la couverture en gras de la face extérieure de la demi-carcasse.

16. Dispositif selon l'une quelconque des revendications 7 à 15, caractérisé en ce que la surface du dièdre est inclinée d'un angle de 0 à 20 degrés par rapport à la verticale, de telle sorte que ses bords haut et bas soient disposés de part et d'autre du plan vertical contenant la chaîne assurant le transport des demi-carcasses.

## Patentansprüche

1. Verfahren für die Klassifizierung von Rümpfen von Großrindern, Kälbern, Schweinen oder Schafen, umfassend das Anlegen eines Halb-Rumpfs mit der der Symmetrieachse des Rumpfs entsprechenden Längsfläche desselben gegen eine Auflage, das Durchführen optischer Aufnahmen unter verschiedenen Winkeln, das Speichern dieser Aufnahmen im Speicher eines Rechners und das Verarbeiten der erfaßten Informationen zum Durchführen der Klassifizierung des Rumpfs,
dadurch gekennzeichnet, daß es umfaßt:
- das zur Anlage bringen des Halb-Rumpfs einerseits mit seiner Längsschnittfläche gegen eine Auflagefläche, welche in einer im wesentlichen vertikalen Ebene angeordnet ist, die im wesentlichen der die Aufhänghaken der Schlachtungskette enthaltenden Ebene entspricht, wobei diese Auflagefläche von einem mit der gleichen Geschwindigkeit wie die Kette angetriebenen Endlosband gebildet ist, und andererseits mit seiner Rückenfläche in der Nähe seines Unterendes gegen eine in Vorrückrichtung der Schlachtungskette in einem stromabwärtigen Endbereich derselben angeordnete zweite Auflagefläche, die durch einen senkrecht zum Band schwenkbaren Flügel gebildet ist,
- das genaue Höhenpositionieren der Band-Flügel-Einheit mittels einer Kamera (L),
- das Anhalten der Kette nachdem der Halb-Rumpf zur Anlage am Flügel gekommen ist,
- das Winkelpositionieren des Rumpfs zum Durchführen optischer Aufnahmen unter verschiedenen Winkeln im Bereich des Hinterteils, des Schenkels, des Oberabschnitts, des oberen Teils des Rückrats und des Vorderteils des Halb-Rumpfs, welcher dem tiefliegenden Bereich desselben entspricht,
- das Speichern der Aufnahmen im Speicher eines Rechners, welcher bereits die Gewichts- und Längenmessungen dieses Halb-Rumpfs enthält,
- das Verarbeiten der erfaßten Informationen unter Berücksichtigung theoretischer Information, um die Klassifizierung der Rümpfe durchzuführen,
- den Flügel in seine den Rumpf freigebende und sein Wegführen erlaubende Stellung klappen zu lassen.

2. Verfahren nach Anspruch 1, dadurch gekennzeichnet, daß es das Durchführen wenigstens einer Aufnahme an der Innenfläche des Rumpfs, das Übertragen dieser Information zum Rechner, und das Verarbeiten dieser Information in Abhängigkeit von den gespeicherten Elementen umfaßt, um den Zustand der Erfassung zu bestimmen.

3. Verfahren nach einem der Ansprüche 1 und 2, dadurch gekennzeichnet, daß es zunächst das Wiegen jedes Halb-Rumpfs umfaßt, dessen Klassifizierung durchzuführen ist, wobei das gemessene Gewicht eine Information über die Länge des Rumpfs liefert, die zum Durchführen einer ersten Einstellung der Höhenposition der Auflage zur Anlage des Halb-Rumpfs verwendet wird.

4. Verfahren nach Anspruch 3, dadurch gekennzeichnet, daß es nach dem Wiegen jedes Halb-Rumpfs eine optische Messung der Länge desselben umfaßt, welche eine zur Durchführung einer genauen Einstellung der Höhenposition der Auflage zur Anlage des Halb-Rumpfs verwendete Information liefert.

5. Verfahren nach Anspruch 1, dadurch gekennzeichnet, daß es ein direktes Beleuchten des Rumpfs (4) von der Aufnahmeseite umfaßt, was es erlaubt, einen Kontrast zwischen dem Rumpf und dem Hintergrund zu erzielen, von dem derselbe sich abhebt.

6. Verfahren nach Anspruch 1, dadurch gekennzeichnet, daß es das Durchführen einer indirekten Beleuchtung des Rumpfs umfaßt, und zwar mittels eines eine diffuse Beleuchtung des ganzen Rumpfs gewährleistenden Leuchthintergrunds.

7. Vorrichtung für die Verwendung des Verfahrens nach Anspruch 1, dadurch gekennzeichnet, daß sie umfaßt:
- ein Anlagesystem für den Halb-Rumpf (4), das die Gestalt eines Dieders aufweist, welcher durch zwei Flächen (7, 9) begrenzt wird, die voneinander durch eine im wesentlichen vertikale Kante getrennt sind, wobei die eine (7) der Flächen des Dieders, die dazu bestimmt ist, gegen die Schnittseite des Halb-Rumpfs zur Anlage zu kommen, durch ein Endlosband (7) gebildet ist, das an vertikalen Achsen (8) angebracht ist, wobei die eine der Seiten des Bandes im wesentlichen in der Ebene der Aufhänghaken (3) der Schlachtungskette angeordnet ist und synchron mit dieser letzteren angetrieben ist, und wobei die andere Seite (9) des Dieders, welche dazu bestimmt ist, gegen die Rückenseite des Halb-Rumpfs in der Nähe des unteren Teils dieses letzteren zur Anlage zu kommen, in der Nähe des in der Fortschrittsrichtung der Schlachtungskette stromabwärtigen Endes des Bands (7) angeordnet ist und durch einen in einer vertikalen Ebene angeordneten Flügel (27) gebildet ist, der zwischen einer Stellung, in der er senkrecht zum Trum der aktiven Seite des Bands ist, und einer Stellung, in der er im wesentlichen in Verlängerung mit diesem ist, schwenkbar ist, wobei der Dieder um eine vertikale Achse herum schwenkbar angebracht ist, welche der Achse des Hakens entspricht, und mit Mitteln zum Drehantrieb verbunden ist, welche nacheinander seine Positionierung in mehreren vorbestimmten Winkelstellungen gewährleisten, die den unterschiedlichen Aufnahmewinkeln entsprechen,
- eine Kamera (L) zum genauen Höhenpositionieren der Band-Flügel-Einheit
- Kameras (C, D, E, G, X), welche an einem festen Träger angebracht sind und dazu bestimmt sind, Ansichten des Halb-Rumpfs im Bereich des Hinterteils, des Schenkels, des oberen Teils des Rückrats und seines Vorderteils aufzunehmen,
- einen Rechner, der dazu bestimmt ist, die Aufnahmen sowie die Messungen der Länge und des Gewichts des Halb-Rumpfs zu speichern und die erfaßten Informationen unter Berücksichtigung theoretischer Informationen zu verarbeiten, um die Klassifizierung der Rümpfe durchzuführen,
- Mittel zum Klappen der zweiten Fläche (9) des Dieders, um den Rumpf freizugeben und sein Wegführen zu erlauben.

8. Vorrichtung nach Anspruch 7, dadurch gekennzeichnet, daß das Band mit einem Waschsystem ausgestattet ist und der Träger des Bands schwenkbar um eine vertikale Achse (23) angebracht ist.

9. Vorrichtung nach Anspruch 7, dadurch gekennzeichnet, daß die Breite des Bands (7), d.h. die Abmessung desselben in Richtung der Höhe betrachtet, deutlich kleiner als die Länge des Rumpfs (4) ist, und der Träger (24) des Bands vertikal verlagerbar angebracht ist.

10. Vorrichtung nach Anspruch 9, dadurch gekennzeichnet, daß die Mittel zum vertikalen Verlagern des Trägers (24) des Bands (7) und des ihm zugeordneten Flügels (27) durch einen Schlitten (19) gebildet sind, an welchem der Träger des Bands und des Flügels schwenkbar angebracht ist, wobei dieser Schlitten selbst an vertikalen Säulen (20) mit vertikalem Antrieb mittels einer Kugelumflaufspindel angebracht ist.

11. Vorrichtung nach Anspruch 7, dadurch gekennzeichnet, daß der Dieder, welcher durch das Band (7) und den Flügel (9, 27) gebildet ist, vor einer Leuchtplatte (5) angeordnet ist, welche eine Höhe aufweist, die im wesentlichen dem Abstand zwischen dem Boden und der die Rümpfe transportierenden Kette entspricht.

12. Vorrichtung nach einem der Ansprüche 7-11, dadurch gekennzeichnet, daß der Vorgang der Vertikalverlagerung des Dieders, der dazu bestimmt ist, die Anlage eines Halb-Rumpfs herzustellen, gesteuert ist einerseits von einer stromaufwärts angeordneten Vorrichtung zum Wiegen des Halb-Rumpfs für eine Grobeinstellung, und andererseits von einer gleichfalls stromaufwärts des Dieders angeordneten optischen Vorrichtung zur Messung der Länge des Rumpfs für eine Feineinstellung derart, daß der Dieder an dem Teil des Rückens des Tiers zur Anlage kommt, welcher oberhalb des Halses desselben gelegen ist.

13. Vorrichtung nach einem der Ansprüche 7-12, dadurch gekennzeichnet, daß der Drehwinkel des Dieders in der Größenordnung von 70 Grad bezüglich der Ursprungsstellung ist, und die Aufnahmen mit wenigstens drei Kameras durchgeführt werden, wobei die optische Achse der ersten (C) orthogonal zur Richtung der Schlachtungskette (2) ist, und wobei die zwei anderen Kameras (D, E), deren optische Achsen in einer horizontalen Ebene enthalten sind, einen Winkel von 45° bilden und symmetrisch bezüglich der ersten Kamera (C) angeordnet sind.

14. Vorrichtung nach einem der Ansprüche 7-13, dadurch gekennzeichnet, daß der Träger des Dieders mit einer Kamera (G) ausgestattet ist, die dazu bestimmt ist, Ansichten des inneren Bereichs des Halb-Rumpfs aufzunehmen, wobei dieser Kamera eine zusätzliche Beleuchtungsvorrichtung zugeordnet ist, und von der Vorrichtung zum Messen der Länge des Rumpfs gesteuert ist.

15. Vorrichtung nach einem der Ansprüche 13-14, dadurch gekennzeichnet, daß sie Kameras mit optischen Achsen umfaßt, welche parallel zu denen der festen Kameras C, D oder E, sind, welche mit zusätzlicher Beleuchtung arbeiten, um die Fettdecke der Außenfläche des Halb-Rumpfs zu untersuchen.

16. Vorrichtung nach einem der Ansprüche 7-15, dadurch gekennzeichnet, daß die Diederfläche mit einem Winkel von 0 bis 20 Grad bezüglich der Vertikalen geneigt ist derart, daß ihr Ober- und Unterrand zu beiden Seiten einer die Kette enthaltenden vertikalen Ebene angeordnet sind, der Kette nämlich, welche den Transport der Halb-Rümpfe gewährleistet.

## Claims

1. Method for classifying carcasses of large cattle, calves, pigs or sheep, consisting of putting a half carcass in abutment, through the longitudinal face thereof corresponding to the axis of symmetry of the carcass, against a support, taking optical photographs from different angles, storing these photographs in the memory of a computer and processing the data captured in order to classify the carcass, characterised in that it consists of:
- bringing the half carcass into abutment, on the one hand, through its longitudinal face, against a support surface situated in a substantially vertical plane corresponding substantially to the vertical plane containing the suspension hooks of the slaughtering line, this support surface consisting of an endless belt driven at the same speed as the line and, on the other hand, through its rear face, close to its bottom end, against a second support surface, disposed at the downstream end thereof, in the direction of movement of the slaughtering line, consisting of a pivoting flap perpendicular to the belt,
- accurately positioning the belt/flap assembly with respect to height by means of a camera (L),
- stopping the line after the half carcass has come to bear on the flap,
- effecting an angular positioning of the carcass in order to take optical photographs from different angles at the hindquarters, the thigh, the upper part of the chine and the front part of the half carcass corresponding to the lower area thereof,
- storing the photographs in the memory of a computer, which already contains the weight and length measurements of this half carcass,
- processing the data captured whilst taking account of theoretical information in order to classify the carcasses, and
- causing the flap to tilt towards its retracted position in order to release the carcass and enable it to be discharged.

2. Method according to Claim 1, characterised in that it consists of taking at least one photograph on the internal face of the carcass, sending this information to the computer and processing this information as a function of stored elements in order to determine the state of recording.

3. Method according to either one of Claims 1 and 2, characterised in that it consists first of all of weighing each half carcass which is to be classified, the measured weight giving information on the length of the carcass, which is used to effect a first adjustment of the position, with respect to height, of the support on which the half carcass bears.

4. Method according to Claim 3, characterised in that it consists, after weighing each half carcass, of taking an optical measurement of the length thereof, which supplies information used in order to effect a precise adjustment of the position, with respect to height, of the support on which the half carcass bears.

5. Method according to Claim 1, characterised in that it consists of directly illuminating the carcass (4) on the same side as the photograph, which makes it possible to obtain contrast between the carcass and the background against which it is standing out.

6. Method according to Claim 1, characterised in that it consists of indirectly illuminating the carcass, by means of a luminous background providing a diffuse illumination of the entire carcass.

7. Device for implementing the method according to Claim 1, characterised in that it comprises:
- a support system for the half carcass (4) in the form of a dihedron delimited by two surfaces (7, 9) separated from each other by a substantially vertical edge, one (7) of the faces of the dihedron designed to bear against the cut face of the half carcass consisting of an endless belt (7) mounted on vertical axes (8), with one of the faces of the belt disposed substantially in the plane of the suspension hooks (3) of the slaughtering line and driven in synchronism with the latter, and the other face (9) of the dihedron designed to bear against the back face of the half carcass, in proximity to the lower part of the latter, being situated in proximity to the downstream end of the belt (7), in the direction of movement of the slaughtering line, consisting of a flap (27) disposed in a vertical plane, mounted so as to pivot between a position in which it is perpendicular to the side of the active face of the belt, and a position in which it is substantially in line therewith, the dihedron being mounted so as to pivot about a vertical axis corresponding to the axis of the hook and associated with means of driving in rotation successively providing its positioning in several predetermined angular positions corresponding to the different photograph angles,
- a camera (L) for accurate height positioning of the belt/flap assembly
- cameras (C, D, E, G, X) mounted on a fixed support and designed to take photographs of the half carcass at the hindquarters, thigh, upper part of the chine and its front part,
- a computer designed to store the photographs, and the measurements of length and weight of the half carcass, and to process the data captured whilst taking account of theoretical information in order to classify the carcasses,
- means of retracting the second face (9) of the dihedron in order to release the carcass and enable it to be discharged.

8. Device according to Claim 7, characterised in that the belt is equipped with a washing system, and the belt support is mounted so as to pivot about a vertical axis (23).

9. Device according to Claim 7, characterised in that the width of the belt (7), that is to say the dimension thereof considered in the direction of the height, is substantially less than the length of the carcass (4), and the belt support (24) is mounted so as to be able to move vertically.

10. Device according to Claim 9, characterised in that the means of vertical movement of the support (24) for the belt (7) and of the flap (27) which is associated therewith consist of a slide (19) on which the support for the belt and flap is mounted in an articulated fashion, this slide itself being mounted on vertical columns (20), with vertical driving by means of a ballscrew.

11. Device according to Claim 7, characterised in that the dihedron formed by the belt (7) and the flap (9, 27) is disposed in front of a luminous panel (5) of height corresponding substantially to the distance between the floor and the line transporting the carcasses.

12. Device according to any one of Claims 7 to 11, characterised in that the vertical displacement movement of the dihedron designed to effect the support of a half carcass is controlled on the one hand by a device for weighing the half carcass disposed upstream for an approximate adjustment and on the other hand by an optical device for measuring the length of the carcass, also disposed upstream of the dihedron, for fine adjustment, so that the dihedron bears on the part of the back of the animal situated above the neck thereof.

13. Device according to any one of Claims 7 to 12, characterised in that the angle of rotation of the dihedron is around 70 degrees with respect to the original position, and the photographs are taken by at least three cameras, where the optical axis of the first (C) is perpendicular to the direction of the slaughtering line (2) and where the other two cameras (D, E), whose optical axes are contained in a horizontal plane, form an angle of 45°, and are disposed symmetrically with respect to the first camera (C).

14. Device according to any one of Claims 7 to 13, characterised in that the support for the dihedron is equipped with a camera (G) which is designed to take photographs of the internal part of the half carcass, this camera being associated with an additional illumination device, and controlled by the device for measuring the length of the carcass.

15. Device according to either one of Claims 13 to 14, characterised in that it comprises cameras with optical axes parallel to those of the fixed cameras C, D or E, functioning with additional illumination, in order to analyse the fat cover of the external face of the half carcass.

16. Device according to any one of Claims 7 to 15, characterised in that the surface of the dihedron is inclined at an angle of 0 to 20 degrees with respect to the vertical, so that its upper and lower edges are disposed on each side of the vertical plane containing the line transporting the half carcasses.
